# EUROPEAN PATENT APPLICATION

(11) **EP 2 248 458 A1**
(43) Date of publication of application: **10.11.2010**
(21) Application number: 08872539.5
(22) Date of filing: 24.12.2008
(51) Int. Cl.: A61B 1/06, G02B 23/26, H01S 3/10

(54) **LIGHTING DEVICE AND ENDOSCOPE SYSTEM**

(30) Priority: 20.02.2008 JP 2008039215
(71) Applicant: Olympus Corporation, Shibuya-ku Tokyo 151-0072 (JP)
(72) Inventor: IMADE, Shinichi, Tokyo 151-0072 (JP)
(74) Representative: Schicker, Silvia
(86) International application number: PCT/JP2008/073380
(87) International publication number: WO 2009/104340

(57) **Abstract**

Provided is an illumination device in which loss of light intensity is small and deterioration of directivity is prevented. Provided is an illumination device including at least one white light source that emits white light; a first light guiding unit that is disposed on an emission optical axis of the white light source and that guides the white light emitted from the white light source outward in a radial direction perpendicular to the emission optical axis; a second light guiding unit that is arrayed with the first light guiding unit in a direction along the emission optical axis and that guides light going inward in the radial direction in the direction along the emission optical axis; a rotating unit that rotationally drives the first light guiding unit and the second light guiding unit together about the emission optical axis; a plurality of semiconductor light sources that are disposed outward of the second light guiding unit in the radial direction and arrayed in a circumferential direction about the emission optical axis and that emit illuminating light inward in the radial direction; and a plurality of wavelength-range selecting units that are disposed outward of the first light guiding unit and the second light guiding unit on opposite sides in the radial direction at circumferential positions different from those of the semiconductor light sources and that select light components in predetermined wavelength ranges from the white light guided by the first light guiding unit and return the light components to the second light guiding unit.

## Description

### Technical Field

The present invention relates to an illumination device and an endoscope system in which semiconductor light sources, such as LEDs, are used to enable adaptive switching of illumination conditions.

### Background Art

With improvements in emission efficiency and practical adoption of high-luminance white-light LEDs and high-luminance RGB primary color light, semiconductor light sources, such as light-emitting diodes (hereinafter referred to as "LEDs") and semiconductor lasers, are being employed as illumination light sources in image display systems such as large-screen liquid crystal television sets or projectors, in which the application of LEDs has been considered difficult because of the insufficient luminance of LEDs. Furthermore, also with light sources for medical applications, such as endoscopes, and with light sources for microscopes, replacement of the conventional xenon lamps, halogen lamps, and metal halide lamps with white LEDs or RGB primary color LEDs has started. These LEDs have features such as long life, low power consumption, ease of intensity control, and low environmental load due to the absence of mercury, and are expected to offer benefits as light sources.

With light sources for medical applications and microscopes, there are cases where illuminating light having various wavelength ranges is required for special-light observation as well as high-luminance white light, and it is necessary to provide illumination while selectively switching among light beams with a plurality of colors. However, generally, only LEDs that have specific wavelength ranges are being manufactured, so that the colors of light that can be obtained are limited. Therefore, it is necessary to extract illuminating light of an arbitrary wavelength range from an existing lamp light source by using a spectrum filter. However, there is a disadvantage that a loss of light intensity occurs when a spectrum filter is used.

Furthermore, as a method of selective switching among colors of light, for example, in a known method, light from a plurality of white light lamps is sequentially turned on in a time-division manner, and a rotating mirror having a reflecting area and a transmitting area on one disk is rotated, whereby switching is performed among the generated light with the plurality of colors (e.g., see Patent Citation 1).
Patent Citation 1: Japanese Unexamined Patent Application, Publication No. 2000-89139

### Disclosure of Invention

With the above technique, however, there is a shortcoming that, with a single rotating mirror, it is possible to combine light from only two light sources into a light path in the same direction. Furthermore, in the case of a method in which the light path is switched by changing the angle of a reflecting mirror, there is a disadvantage that the light flux is directed in an incorrect direction in a transient state of switching, causing a loss of light intensity. Furthermore, in the case where reflection by a mirror is employed, there is a disadvantage that the reflection has an effect of widening the angle of light from a light source, causing reduced directivity.

The present invention has been made in view of the situation described above, and it is an object thereof to provide an illumination device and an endoscope system in which switching among light paths of illuminating light from a plurality of light emission sources is performed to guide the illuminating light in the same direction so that loss of light intensity is reduced and reduction of directivity is prevented.

In order to achieve the above object, the present invention employs the following solutions.
A first aspect of the present invention is an illumination device including at least one white light source that emits white light; a first light guiding unit that is disposed on an emission optical axis of the white light source and that guides the white light emitted from the white light source outward in a radial direction perpendicular to the emission optical axis; a second light guiding unit that is arrayed with the first light guiding unit in a direction along the emission optical axis and that guides light going inward in the radial direction in the direction along the emission optical axis; a rotating unit that rotationally drives the first light guiding unit and the second light guiding unit together about the emission optical axis; a plurality of semiconductor light sources that are disposed outward of the second light guiding unit in the radial direction and arrayed in a circumferential direction about the emission optical axis and that emit illuminating light inward in the radial direction; and a plurality of wavelength-range selecting units that are disposed outward of the first light guiding unit and the second light guiding unit on opposite sides in the radial direction at circumferential positions different from those of the semiconductor light sources and that select light components in predetermined wavelength ranges from the white light guided by the first light guiding unit and return the light components to the second light guiding unit.

According to the first aspect of the present invention, the rotating unit rotationally drives the first light guiding unit and the second light guiding unit together. When a wavelength-range selecting unit is located radially outward of the first light guiding unit and the second light guiding unit, white light emitted from the white light source is guided to the wavelength-range selecting unit by the first light guiding unit, the wavelength-range selecting unit selects light components in its predetermined wavelength range, and the light components are emitted by the second light guiding unit in the direction along the emission optical axis of the white light source. On the other hand, when a semiconductor light source is located radially outward of the first light guiding unit and the second light guiding unit, illuminating light emitted from the semiconductor light source is emitted by the second light guiding unit in the same direction as light from the white light source. That is, by the operation of the rotating unit, it is possible to emit light components in a specific wavelength range selected from white light and illuminating light from the semiconductor light sources in the same direction sequentially by switching. Note that since it is possible to emit light with a large current instantaneously by synchronizing the pulsed lighting of the semiconductor light sources and the position of the first light guiding unit and the second light guiding unit, it is possible to emit illuminating light with a high luminance.

In the above first aspect, the white light source may be a xenon lamp, a halogen lamp, a metal halide lamp, or an ultra-high-pressure mercury lamp.
Accordingly, it is possible to obtain light components in a specific wavelength range with a high luminance.

In the above first aspect, the wavelength-range selecting units may each include a first right-angled prism that reflects the white light guided by the first light guiding unit in a direction substantially parallel to the emission optical axis; a second right-angled prism that reflects the light reflected by the first right-angled prism inward in the radial direction; and a wavelength-range selecting filter that passes only light components in the predetermined wavelength range.
Accordingly, it becomes possible to return the white light going radially outward from the first light guiding unit radially inward with the first right-angled prism and the second right-angled prism and to cause light components in a specific wavelength range to become incident on the second light guiding unit in accordance with the type of wavelength-range selecting filter.

In the above first aspect, the first light guiding unit may include a right-angled prism that reflects the white light emitted from the white light source outward in the radial direction; and a parallel rod that guides the white light reflected by the right-angled prism to the wavelength-range selecting units.
Accordingly, it is possible to guide the white light emitted from the white light source to the wavelength-range selecting units while preventing loss of light intensity and improving directivity.

In the above first aspect, the second light guiding unit may includes a parallel rod that guides the light components returned by the wavelength-range selecting units and the illuminating light emitted from the semiconductor light sources inward in the radial direction; and a right-angled prism that reflects the light components and illuminating light guided by the parallel rod in the direction along the emission optical axis.
Accordingly, it is possible to emit the light components returned from the wavelength-range selecting units and the illuminating light emitted from the semiconductor light sources in the direction along the emission optical axis while preventing loss of light intensity and improving directivity.

In the above first aspect, there may be further provided an illumination-mode selecting unit that enables selection of an illumination mode and a controller that controls the turn-on and turn-off timing and light intensities of the semiconductor light sources, the turn-on and turn-off timing and light intensity of the white light source, and the operation of the rotating unit by different methods based on the illumination mode selected.
Accordingly, it is possible to emit the illuminating light emitted from the semiconductor light sources and light components in a specific wavelength range selected by a wavelength-range selecting unit in accordance with the illumination mode selected.

In the above first aspect, in accordance with the illumination mode selected, the controller may cause the rotating unit to rotate at a constant speed and cause the semiconductor light source facing the entrance end of the second light guiding unit to turn on.
Accordingly, it is possible to emit illuminating light with a high luminance by synchronizing the phase of the rotating unit with the semiconductor light sources and turning on the semiconductor light source facing the entrance end of the second light guiding unit in a pulsed manner.

In the above first aspect, in accordance with the illumination mode selected, the controller may stop the entrance end of the second light guiding unit at a position facing the wavelength-range selecting unit whose predetermined wavelength range includes all components of the white light.
Accordingly, all the components of white light become incident from the wavelength-range selecting unit to the entrance end of the second light guiding unit, so that it is possible to emit white light with a high luminance.

In the above first aspect, in accordance with the illumination mode selected, the controller may stop the entrance end of the second light guiding unit at a position facing the wavelength-range selecting unit or semiconductor light source corresponding to a specific color.
Accordingly, light components or illuminating light in a wavelength range corresponding to the specific color become incident on the entrance end of the second light guiding unit, so that it is possible to emit light in the specific color.

In the above first aspect, the controller may change the position of the second light guiding unit relative to the wavelength-range selecting units or the semiconductor light sources to adjust the intensity of light captured by the second light guiding unit.
Accordingly, it is possible to adjust the intensity of the illuminating light emitted from the semiconductor light sources and light components in a specific wavelength range selected by a wavelength-range selecting unit.

In the above first aspect, there may be further provided a light detecting unit that detects the intensity of the white light guided by the first light guiding unit; and a fail-safe monitoring controller that detects an abnormality of the white light source based on the intensity of the white light detected by the light detecting unit and, when an abnormality is detected, that keeps the semiconductor light sources constantly turned on and causes the entrance end of the second light guiding unit to face the semiconductor light sources.
Accordingly, with the fail-safe monitoring controller, it is possible to detect an abnormality of the white light source based on a value detected by the light detecting unit and, when an abnormality is detected, to select and emit the illuminating light from the semiconductor light sources.

A second aspect of the present invention is an endoscope system including the illumination device described above, including a light guiding member that guides illuminating light emitted by the illumination device to an observed area; an image capturing element that acquires an image of the observed area irradiated with the illuminating light guided by the light guiding member; and a display that displays the image acquired by the image capturing element.
According to the second aspect of the present invention, it is possible to irradiate an observed area via the light guiding member with light components or illuminating light in a wavelength range suitable for the observed area or symptoms and to acquire an image of the observed area with the image capturing element and display the image on the display. This makes it possible to improve the accuracy of special-light observation and the working efficiency.

According to the present invention, by performing switching sequentially among light paths of illuminating light from a plurality of light emitting sources and guiding light in the same direction by using a rod optical system, advantages are afforded in that loss of light intensity is small and deterioration of directivity is prevented.

### Brief Description of Drawings

[FIG. 1A] Fig. 1A is a schematic illustration for explaining the configuration of an illumination device according to a first embodiment of the present invention, showing a longitudinal sectional view taken in a direction along the emission optical axis of a white light lamp.
[FIG. 1B] Fig. 1B is a schematic illustration for explaining the configuration of the illumination device according to the first embodiment of the present invention, showing a cross sectional view taken in a radial direction perpendicular to the emission optical axis of the white light source.
[FIG. 2A] Fig. 2A is a schematic illustration for explaining the operation of the illumination device in Fig. 1A.
[FIG. 2B] Fig. 2B is a schematic illustration for explaining the operation of the illumination device in Fig. 1B.
[FIG. 3] Fig. 3 is a partial enlarged view for explaining the configuration of a basic component of semiconductor light source units in Figs. 1A and 1B.
[FIG. 4] Fig. 4 is a diagram showing an example relationship between individual light sources of the illumination device in Figs. 1A and 1B and the spectrum.
[FIG. 5] Fig. 5 is a control block diagram of the illumination device in Figs. 1A and 1B.
[FIG. 6] Fig. 6 is a schematic illustration for explaining the rotation angle of light guiding units in the illumination device in Figs. 1A and 1B.
[FIG. 7] Fig. 7 is a chart showing the lighting sequence of the light source units in Figs. 1A and 1B.
[FIG. 8A] Fig. 8A shows the illumination device in Fig. 1B as a comparative example.
[FIG. 8B] Fig. 8B is a schematic illustration for explaining the configuration of an illumination device according to a second embodiment of the present invention, which is a schematic illustration for explaining the configuration in a white-light illumination mode.
[FIG. 9] Fig. 9 is a schematic illustration for explaining the operation of the illumination device in Fig. 8B.
[FIG. 10] Fig. 10 is a graph for explaining the relationship between the intensity of light emitted and the rotation angle of light guiding units in the illumination device in Fig. 8B.
[FIG. 11A] Fig. 11A shows the illumination device in Fig. 1B as a comparative example.
[FIG. 11B] Fig. 11B is a schematic illustration for explaining the configuration of the illumination device in Fig. 8B, which is a schematic illustration for explaining the configuration in a specific-color illumination mode.
[FIG. 12] Fig. 12 is a flowchart for explaining control in individual illumination modes of the illumination device in Fig. 8B.
[FIG. 13A] Fig. 13A is a schematic illustration for explaining the configuration of an illumination device according to a third embodiment of the present invention, showing a longitudinal sectional view taken along the emission optical axis of a white light lamp.
[FIG. 13B] Fig. 13B is a schematic illustration for explaining the configuration of the illumination device according to the third embodiment of the present invention, showing a cross sectional view taken in a radial direction perpendicular to the emission optical axis of the white light source.
[FIG. 14] Fig. 14 is a chart for explaining the lighting sequence of light source units in Figs. 13A and 13B.
[FIG. 15] Fig. 15 is a control block diagram of an illumination device according to a fourth embodiment of the present invention.
[FIG. 16] Fig. 16 is a flowchart for explaining control of the illumination device in Fig. 15.
[FIG. 17A] Fig. 17A is a schematic illustration for explaining the configuration of an illumination device according to a fifth embodiment of the present invention, showing a longitudinal sectional view taken along the emission optical axis of a white light lamp.
[FIG. 17B] Fig. 17B is a schematic illustration for explaining the configuration of the illumination device according to the fifth embodiment of the present invention, showing a cross sectional view taken in a radial direction perpendicular to the emission optical axis of the white light source.
[FIG. 18A] Fig. 18A is a schematic illustration for explaining the operation of the illumination device in Fig. 17A.
[FIG. 18B] Fig. 18B is a schematic illustration for explaining the operation of the illumination device in Fig. 17B.
[FIG. 19] Fig. 19 is a partial enlarged view for explaining the configuration of a basic component of wavelength conversion units in Fig. 17.

### Explanation of Reference:

1, 2, 3, 4, 5: Illumination device
11: White light lamp
12: First light guiding unit
13: Second light guiding unit
14: Rotating unit
15, 71: Semiconductor light source unit
16: Wavelength-range selecting unit
17: Emission rod
21, 25: Right-angled prism
22, 24: Parallel rod
27, 75: First right-angled prism
28, 77: Second right-angled prism
29: Wavelength-range selecting filter
32: LED
41: Rotating rod
42: Rotating motor
43: Rotating-rod holding member
44: Rotation sensor
52: Illumination-mode selecting unit
53: System controller
62: Fail-safe monitoring controller
63: Light sensor
72: Wavelength conversion unit
76: Phosphor
81: Dichroic filter
O: Emission optical axis

### Best Mode for Carrying Out the Invention

### [First Embodiment]

Now, an illumination device according to a first embodiment of the present invention will be described with reference to the drawings.

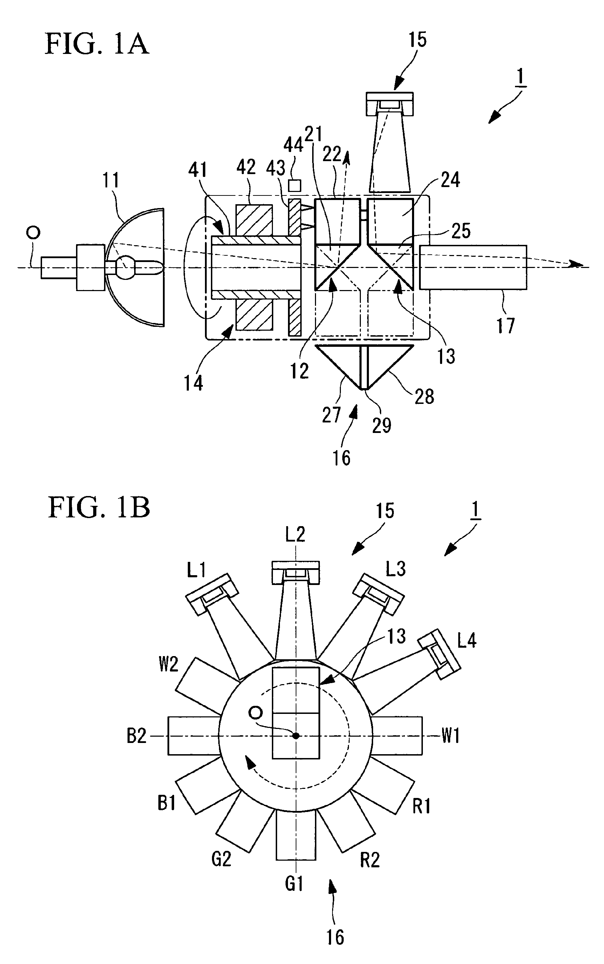

Figs. 1A and 1B are schematic illustrations for explaining the illumination device according to this embodiment. Fig. 1A is a longitudinal sectional view taken in the direction along the emission optical axis of a white light lamp, and Fig. 1B is a cross sectional view taken in a radial direction perpendicular to the emission optical axis of the white light source.

As shown in Fig. 1A, an illumination device 1 according to this embodiment includes a white light lamp (white light source) 11 that emits white light, a first light guiding unit 12 and a second light guiding unit 13 arrayed in the direction along the emission optical axis O of the white light lamp 11, a rotating unit 14 that is connected to the first light guiding unit 12 and the second light guiding unit 13 and that rotates these light guiding units together about the emission optical axis O, a plurality of semiconductor light source units 15 disposed radially outward of the second light guiding unit 13 and arrayed in the circumferential direction about the emission optical axis O, a plurality of wavelength-range selecting units 16 disposed radially outward on opposite sides of the first light guiding unit 12 and the second light guiding unit 13 at circumferential positions different from those of the semiconductor light source units 15, and an emission rod 17 disposed adjacent to the second light guiding unit 13 in the direction along the emission optical axis O.

The white light lamp 11 is, for example, a xenon lamp, a halogen lamp, a metal halide lamp, an ultra-high-pressure mercury lamp, or the like. At least one white light lamp 11 is disposed with its emission optical axis O oriented toward the first light guiding unit 12 to emit white light.

The first light guiding unit 12 includes a right-angled prism 21 that reflects the white light emitted from the white light lamp 11 and deflects the white light by 90° and a parallel rod 22 that guides the white light reflected by the right-angled prism 21 in a radially outward direction.
The second light guiding unit 13 includes a parallel rod 24 that guides light coming in from a radially outward direction in a radially inward direction and a right-angled prism 25 that reflects the light guided by the parallel rod 24 and deflects the light by 90° in the direction along the emission optical axis O.

The rotating unit 14 is disposed along the emission optical axis O of the white light lamp 11 and includes a cylindrical rotating rod 41 having a penetrating hole for passing white light, a rotating motor 42 that rotationally drives the rotating rod 41 about the emission optical axis O, a rotating-rod holding member 43 that secures the rotating rod 41 and the first light guiding unit 12 together, and a rotation sensor 44 that detects the phase of rotation of the rotating-rod holding member 43.

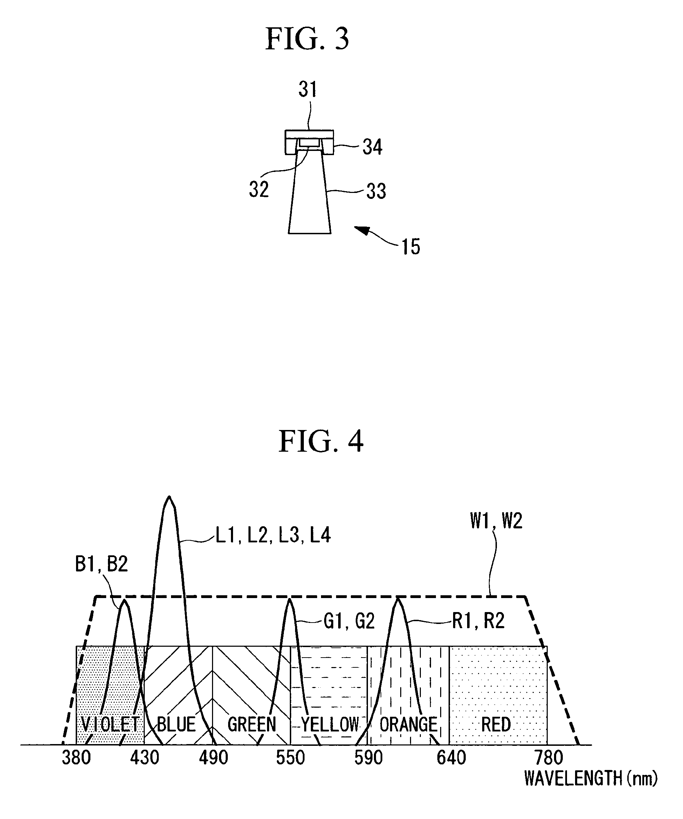

As shown in Fig. 3, the semiconductor light source units 15 each include a substrate 31, an LED (semiconductor light source) 32 that is fixed on the substrate 31 and that emits illuminating light, a tapered rod 33 that guides the illuminating light emitted from the LED 32 in a radially inward direction, and a reflecting member 34 that reflects the illuminating light emitted from the LED 32 so that the illuminating light is incident on the tapered rod 33.
Furthermore, as shown in Fig. 1B, the semiconductor light source units 15 are arrayed at four positions (reference signs L1, L2, L3, and L4) in the circumferential direction about the emission optical axis O.

The wavelength-range selecting units 16 each include a first right-angled prism 27 that reflects the white light guided in the radially outward direction by the first light guiding unit 12 in a direction substantially parallel to the emission optical axis O, a second right-angled prism 28 that reflects the light reflected by the first right-angled prism 27 in a radially inward direction, and a wavelength-range selecting filter 29 that is disposed between the first right-angled prism 27 and the second right-angled prism 28 and that passes only light components in a predetermined wavelength range. Thus, the wavelength-range selecting units 16 are configured to each select light components in the predetermined wavelength range while returning the white light guided in the radially outward direction in the radially inward direction. Furthermore, as shown in Fig. 1B, the wavelength-range selecting units 16 are arrayed at eight positions (reference signs W1, W2, R1, R2, G1, G2, B1, and B2) in the circumferential direction about the emission optical axis O and include wavelength-range selecting filters 29 with different passing wavelength ranges. The wavelength-range selecting units 16 corresponding to the reference signs W1 and W2 may include wavelength-range selecting filters 29 that pass light of all wavelength ranges, but need not include any wavelength-range selecting filters 29.

The emission rod 17 is, for example, a parallel rod whose entrance end and exit end have the same area, and it is configured so that light guided in the direction along the emission optical axis O by the second light guiding unit 13 is further emitted in the direction along the emission optical axis O.

Now, an example of the relationship between the individual light sources and the spectrum will be described using Fig. 4.
As shown in Fig. 4, the wavelength-range selecting units 16 corresponding to the reference signs B1 and B2 are configured to pass, for example, only violet light components, which fall in a wavelength range of about 400 nm to about 450 nm. The wavelength-range selecting units 16 corresponding to the reference signs G1 and G2 are configured to pass, for example, only yellowish-green light components, which fall in a wavelength range of about 520 nm to about 570 nm. The wavelength-range selecting units 16 corresponding to the reference signs R1 and R2 are configured to pass, for example, only orange light components, which fall in a wavelength range of about 580 nm to about 630 nm.
Furthermore, the semiconductor light source units 15 denoted by the reference signs L1, L2, L3, and L4 are configured to emit blue illuminating light, which fall in a wavelength range of about 400 nm to about 500 nm, emitting illuminating light at a high luminance by means of pulsed lighting.

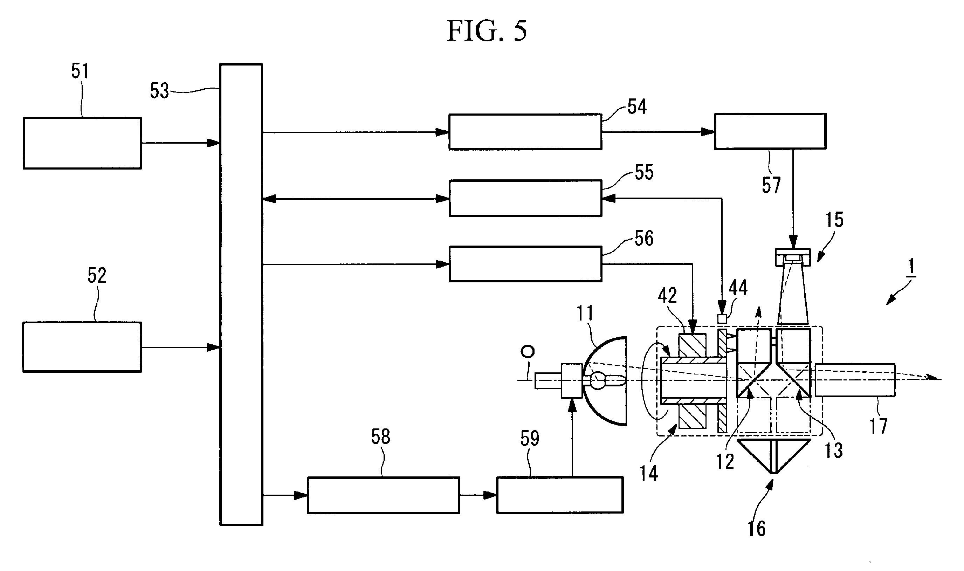

Next, control of the thus-configured illumination device 1 will be described using Fig. 5.
A system controller (controller) 53 controls an LED lighting controller 54, a rotating-motor driver 56, and a lamp lighting controller 58 based on signals output from an illumination-device driving instruction unit 51, an illumination-mode selecting unit 52, and a rotation-sensor driver 55.

The illumination-mode selecting unit 52 is configured so that a user is allowed to select a multiband illumination mode, in which illumination is provided by sequentially switching among light components in various wavelength ranges, a white-light illumination mode, in which white light is used for illumination, or a specific-color illumination mode, in which light components in a wavelength range corresponding to a specific color are used for illumination.
The illumination-device driving instruction unit 51 is, for example, an illumination switch, and it is configured to instruct the system controller 53 that the illumination device 1 be driven in the illumination mode selected by the illumination-mode selecting unit 52.
The rotation-sensor driver 55 is configured to drive the rotation sensor 44 based on a control signal from the system controller 53 and to output a detection signal output from the rotation sensor 44 to the system controller 53.

The LED lighting controller 54 is configured to control the illuminating light from the semiconductor light source units 15 via an LED driver 57 that drives the semiconductor light source units 15. Specifically, the LED lighting controller 54 controls the electric power that is supplied to the LEDs 32 of the individual semiconductor light source units 15 to control the turn-on and turn-off timing and light intensities of the individual LEDs 32.

The lamp lighting controller 58 is configured to control the white light emitted from the white light lamp 11 via a lamp driver 59 that drives the white light lamp 11. Specifically, the lamp lighting controller 58 controls the electric power that is supplied to the white light lamp 11 to control the turning-on and turning-off of the white light lamp 11.

The rotating-motor driver 56 is configured to control the operation of the rotating motor 42 based on a control signal from the system controller 53. The rotating-motor driver 56 is configured to control the rotation and stopping of the rotating motor 42 and to control the rotation phase by using a known control method.

Next, the lighting method of the thus-configured illumination device 1 will be described below.
Here, the case where the multiband illumination mode is selected by the illumination-mode selecting unit 52 will be described.
Upon receiving an instruction for driving the illumination device 1 issued by the illumination-device driving instruction unit 51, the system controller 53 outputs to the rotating-motor driver 56 a control signal that causes the rotating motor 42 to rotate at a constant speed and detects the rotation phase of the rotating-rod holding member 43, i.e., the rotation phase of the first light guiding unit 12 and the second light guiding unit 13, via the rotation-sensor driver 55.

Based on the rotation phase detected, the system controller 53 controls the semiconductor light source units 15 or the white light lamp 11.
That is, in the case where the second light guiding unit 13 faces a semiconductor light source unit 15, the system controller 53 calculates which semiconductor light source unit 15 it faces and controls the LED lighting controller 54 so that illuminating light is emitted toward the calculated semiconductor light source unit 15. Based on the control signal input thereto, the LED lighting controller 54 supplies electric power to the semiconductor light source unit 15 via the LED driver 57.
Thus, as shown in Fig. 1, illuminating light is emitted from the semiconductor light source unit 15, and the illuminating light is guided in the direction along the emission optical axis O by the second light guiding unit 13 and the emission rod 17.

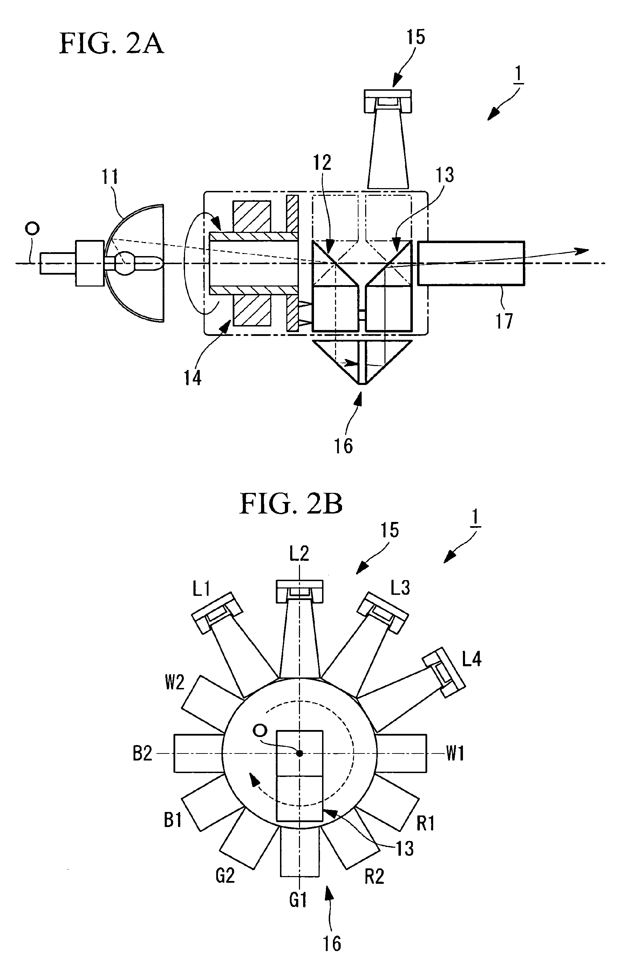

Furthermore, the system controller 53 controls the lamp lighting controller 58 so that the white light lamp 11 emits white light. Based on the control signal input thereto, the lamp lighting controller 58 supplies electric power to the white light lamp 11 via the lamp driver 59.
Thus, as shown in Fig. 2, the white light lamp 11 emits white light, and the white light is guided to a wavelength-range selecting unit 16 by the first light guiding unit. Then, the wavelength-range selecting unit 16 selects light components in the predetermined wavelength range, and the light components are guided in the direction along the emission optical axis O by the second light guiding unit 13 and the emission rod 17.

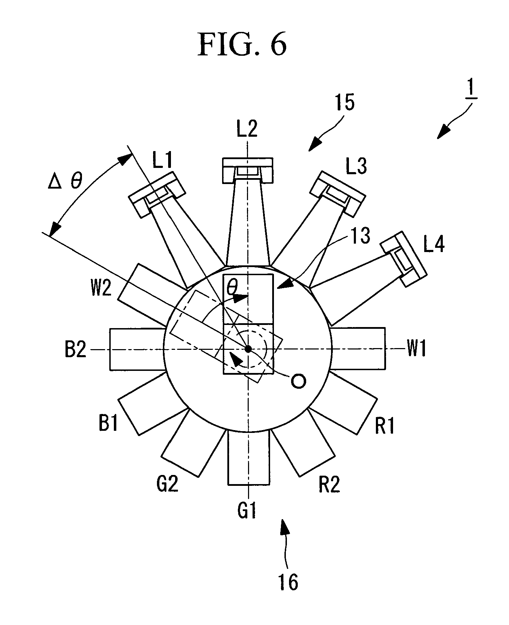

Next, the lighting sequence of the semiconductor light source units 15 and the white light lamp 11 by the system controller 53 will be described.
As shown in Fig. 6, for example, the angle of clockwise rotation of the first light guiding unit 12 and the second light guiding unit 13 about the emission optical axis O from, for example, the position facing the wavelength-range selecting unit 16 corresponding to the reference sign W2 is denoted by θ, and an angle of rotation to the position facing the adjacent semiconductor light source unit 15 or wavelength-range selecting unit 16 is denoted by Δθ.

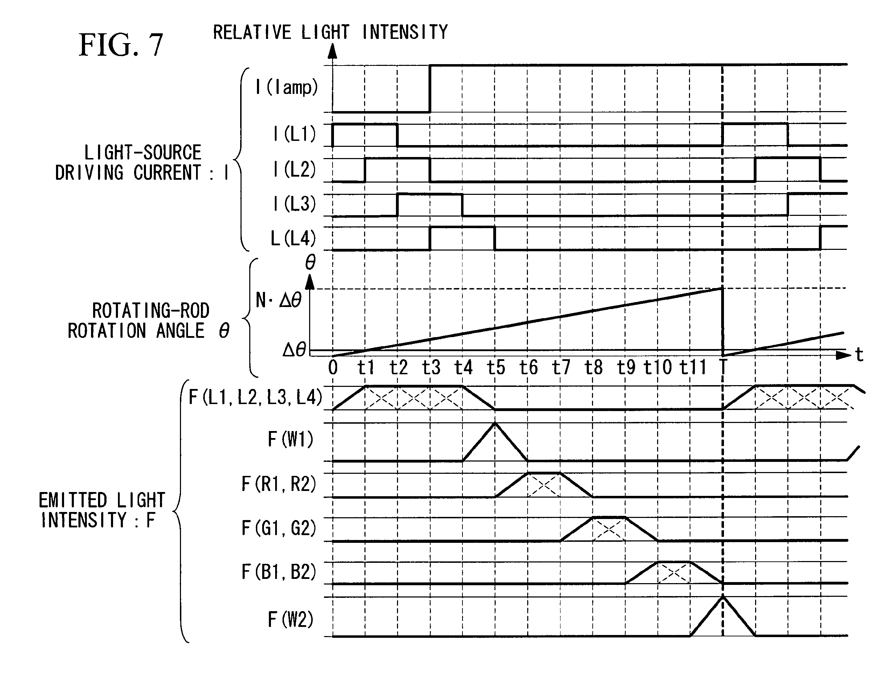

In this case, the lighting sequence of the semiconductor light source units 15 and the white light lamp 11 is expressed as shown in Fig. 7.
In Fig. 7, I denotes the driving current of each light source; that is, I(lamp) denotes the driving current of the white light lamp 11, and I(L1) to I(L4) denote the driving currents of the semiconductor light source units 15 corresponding to the reference signs L1 to L4, respectively. Furthermore, the emission intensity F denotes the intensity of light emitted from the emission rod 17, and F(L1, L2, L3, L4) denotes the intensity of illuminating light emitted from the semiconductor light source units 15 corresponding to the reference signs L1, L2, L3, and L4, and F(W1) and F(W2) denote the intensities of light components that have passed through the wavelength-range selecting units 16 corresponding to the reference signs W1 and W2, respectively. Furthermore, the horizontal axis represents time, and the time at which the first light guiding unit 12 and the second light guiding unit 13 come to the position facing the wavelength-range selecting unit 16 corresponding to the reference sign W2 is denoted by 0, and the time at which the first light guiding unit 12 and the second light guiding unit 13 return to the position facing the wavelength-range selecting unit 16 corresponding to the reference sign W2 is denoted by T.

As shown in Fig. 7, when the first light guiding unit 12 and the second light guiding unit 13 are at the position facing the wavelength-range selecting unit 16 corresponding to the reference sign W1, i.e., at time 0, a driving current I(L1) flows. Then, as the first light guiding unit 12 and the second light guiding unit 13 rotate by Δθ, driving currents I(L2) to I(L4) flow sequentially. In this manner, the semiconductor light source units 15 denoted by the reference signs L1 to L4 are sequentially turned on in a pulsed manner, so that the intensity F(L1, L2, L3, L4) of light emitted from the emission rod 17 is constant.

Then, when the first light guiding unit 12 and the second light guiding unit 13 come to the position facing the semiconductor light source unit 15 corresponding to the reference sign L3, i.e., at time t3, a driving current I(lamp) flows, whereby the white light lamp 11 emits white light. Then, when the first light guiding unit 12 and the second light guiding unit 13 come to the position facing the wavelength-range selecting unit 16 corresponding to the reference sign W1, i.e., at time t5, the intensity of the white light that has passed through the wavelength-range selecting unit 16 corresponding to the reference sign W1 reaches a peak. Then, as the first light guiding unit 12 and the second light guiding unit 13 rotate by Δθ, light components that have passed through the wavelength-range selecting units 16 corresponding to the reference signs R1 to W2 are sequentially emitted from the emission rod 17.

As described above, with the illumination device 1 according to this embodiment, the rotating unit 14 rotationally drives the first light guiding unit 12 and the second light guiding unit 13 together. When one of the wavelength-range selecting units 16 is located radially outward of the first light guiding unit 12 and the second light guiding unit 13, white light emitted from the white light lamp 11 is guided to the wavelength-range selecting unit 16 by the first light guiding unit 12, the wavelength-range selecting unit 16 selects light components in the predetermined wavelength range, and the light components are emitted in the direction along the emission optical axis O of the white light lamp 11 by the second light guiding unit 13. On the other hand, when a semiconductor light source unit 15 is located radially outward of the second light guiding unit 13, illuminating light emitted from the semiconductor light source unit 15 is emitted in the same direction by the second light guiding unit 13 as the light from the white light lamp 11. That is, by the operation of the rotating unit 14, it is possible to emit light components in a specific wavelength range selected from white light and the illuminating light from the semiconductor light source unit 15 in the same direction while sequentially switching between the light components and the illuminating light. Note that it is possible to emit illuminating light having a high luminance by synchronizing the pulsed lighting by the semiconductor light source units 15 and the positions of the first light guiding unit 12 and the second light guiding unit 13.

Furthermore, with the illumination-mode selecting unit 52, which enables selection of an illumination mode, and with the system controller 53, which controls the turn-on and turn-off timing and light intensities of the semiconductor light source units 15, the turn-on and turn-off timing and light intensity of the white light lamp 11, and the operation of the rotating unit 14 by different methods depending on the illumination mode selected, it is possible to emit illuminating light emitted from the semiconductor light source units 15 and light components in a specific wavelength range selected by one of the wavelength-range selecting units 16 in accordance with the illumination mode selected.

Furthermore, in the case where the multiband illumination mode is selected, the system controller 53 causes the rotating unit 14 to rotate at a constant speed and turns on the semiconductor light source unit 15 facing the entrance end of the second light guiding unit 13. Accordingly, the phase of the rotating unit 14 is synchronized with the semiconductor light source units 15, and the semiconductor light source unit 15 facing the entrance end of the second light guiding unit 13 is turned on in a pulsed manner, whereby it is possible to emit illuminating light having a high luminance.

### [Second Embodiment]

Next, an illumination device according to a second embodiment of the present invention will be described with reference to the drawings.
The illumination device according to this embodiment differs from the first embodiment in that the first light guiding unit and the second light guiding unit are stopped at a position in accordance with the selected illumination mode instead of being rotated at a constant speed. Hereinafter, regarding the illumination device according to this embodiment, a description of commonalities with the first embodiment will be omitted, and the description will be directed mainly to differences.

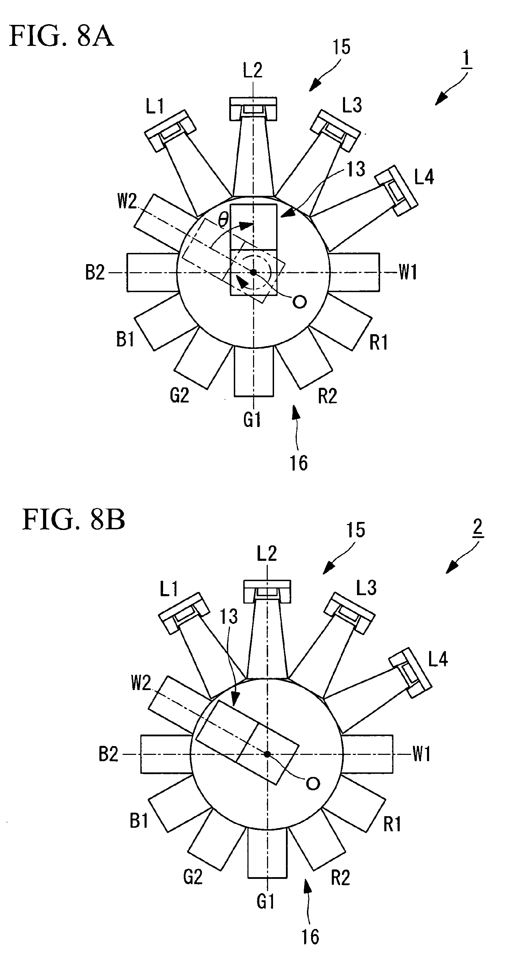

As shown in Fig. 8B, in an illumination device 2 according to this embodiment, the entrance end of the second light guiding unit 13 is stopped at the positions facing the wavelength-range selecting units 16 corresponding to the reference signs W1 and W2 so that all the components of the white light from the white light lamp 11 are emitted in the direction along the emission optical axis O (white-light illumination mode). Note that the illumination device 1 according to the first embodiment is shown in Fig. 8A as a comparative example.

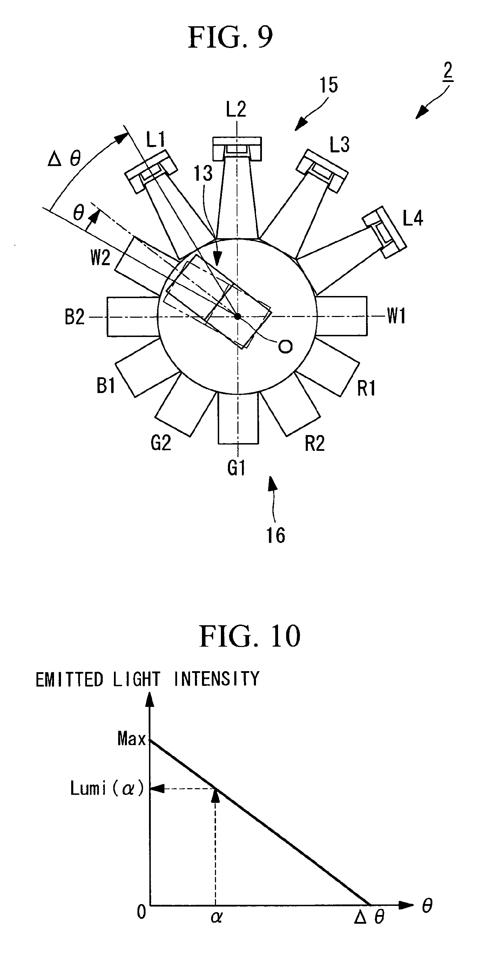

Furthermore, as shown in Fig. 9, in the illumination device 2, the second light guiding unit 13 is rotated as needed from the position facing the wavelength-range selecting unit 16 corresponding to the reference sign W2 by θ° about the emission optical axis O to change the relative positions of the second light guiding unit 13 and the wavelength-range selecting unit 16 corresponding to the reference sign W2.

Here, as shown in Fig. 10, the intensity of light emitted from the emission rod 17 decreases as the angle θ increases. That is, the intensity of light emitted from the emission rod 17 is proportional to the area of the entrance end of the second light guiding unit 13 facing the wavelength-range selecting unit 16 corresponding to the reference sign W2. Therefore, with the illumination device 2 according to this embodiment, it is possible to adjust the intensity of light emitted from the emission rod 17 by rotating the second light guiding unit 13 as needed about the emission optical axis O.

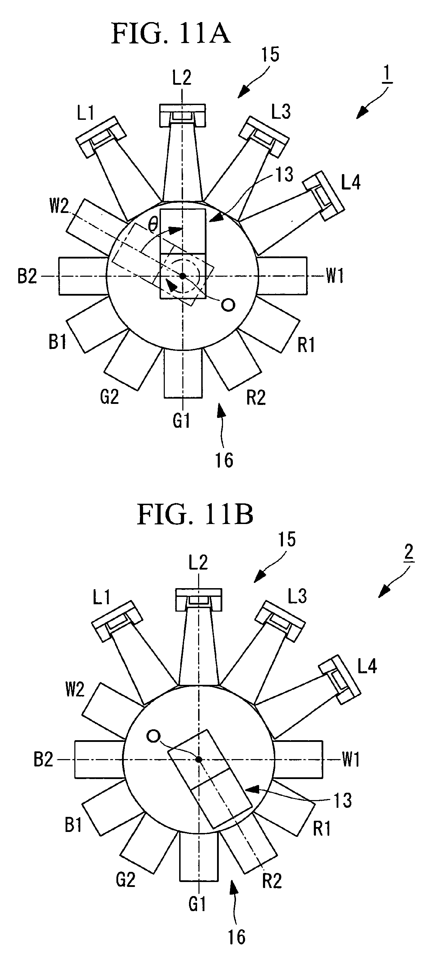

Furthermore, as shown in Fig. 11B, by stopping the entrance end of the second light guiding unit 13 at the position facing the semiconductor light source unit 15 or the wavelength-range selecting unit 16 corresponding to a specific color, it is possible to emit light components in a wavelength range corresponding to the specific color in the direction along the emission optical axis O (specific-color illumination mode). Here, an example where the wavelength-range selecting unit 16 corresponding to the reference sign R2 is selected is shown. Note that shown in Fig. 11A is the illumination device 1 according to the first embodiment as a comparative example. Accordingly, light components or illuminating light in the wavelength range corresponding to the specific color are radiated on the entrance end of the second light guiding unit 13, so that it is possible to emit light of the specific color from the emission rod 17.

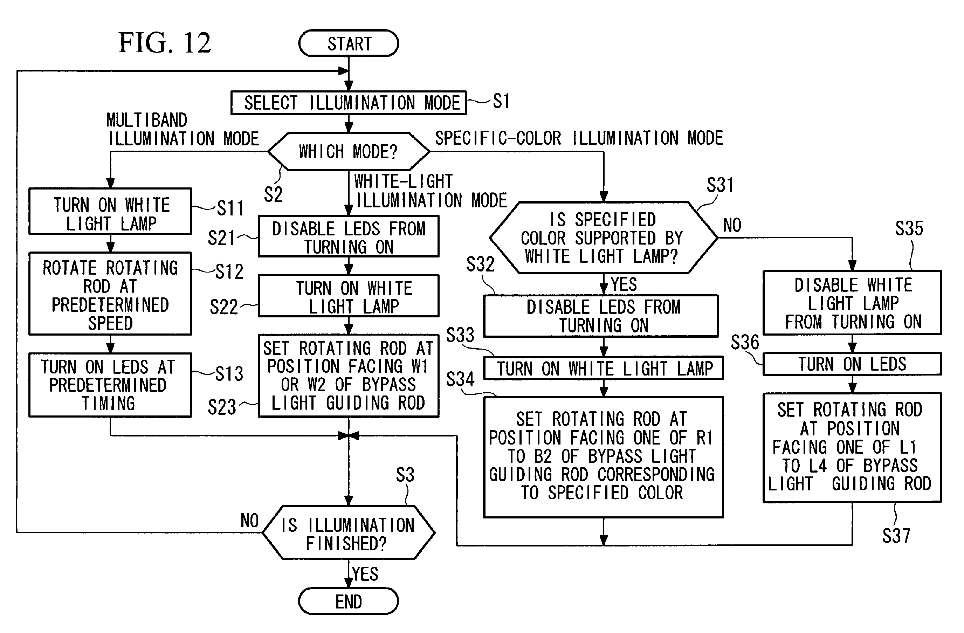

Next, Fig. 12 shows the control flow of the system controller 53 in the individual illumination modes.
As shown in Fig. 12, an illumination mode is selected by a user's operation (S1), and the selected illumination mode is determined (S2).
In the case where the multiband illumination mode is selected, the white light lamp 11 is turned on (S11), the first light guiding unit 12 and the second light guiding unit 13 are rotated at a predetermined speed (S12), and the individual semiconductor light source units 15 are turned on at predetermined timing (S13).

In the case where the white-light illumination mode is selected, the semiconductor light source units 15 are disabled from turning on (S21), the white light lamp 11 is turned on (S22), and the first light guiding unit 12 and the second light guiding unit 13 are stopped at the position facing the wavelength-range selecting unit 16 corresponding to the reference sign W1 or W2 (S23).

In the case where the specific-color illumination mode is selected, it is determined whether the specified color is a color supported by the white light lamp 11 (S31). If the specified color is a color supported by the white light lamp 11, the semiconductor light source units 15 are disabled from turning on (S32), the white light lamp 11 is turned on (S33), and the first light guiding unit 12 and the second light guiding unit 13 are stopped at the position facing the wavelength-range selecting unit 16 corresponding to the specified color (S34). On the other hand, if the specified color is not a color supported by the white light lamp 11 in S31, the white light lamp 11 is disabled from turning on (S35), the semiconductor light source units 15 are turned on (S36), and the first light guiding unit 12 and the second light guiding unit 13 are stopped at the positions facing the semiconductor light source units 15 denoted by the reference signs L1 to L4 (S37).

Through the control described above, it is possible to emit illuminating light from the semiconductor light source units 15 and light components in specific wavelength ranges that have passed through the wavelength-range selecting units 16 in the direction along the emission optical axis O in accordance with the selected illumination mode (S3).

### [Third Embodiment]

Next, an illumination device according to a third embodiment of the present invention will be described with reference to the drawings.
The illumination device according to this embodiment differs from the above-described embodiments in that wavelength-range selecting units corresponding to the reference signs W1 and W2 are not provided. Hereinafter, regarding the illumination device according to this embodiment, a description of commonalities with the above-described embodiments will be omitted, and the description will be directed mainly to differences.

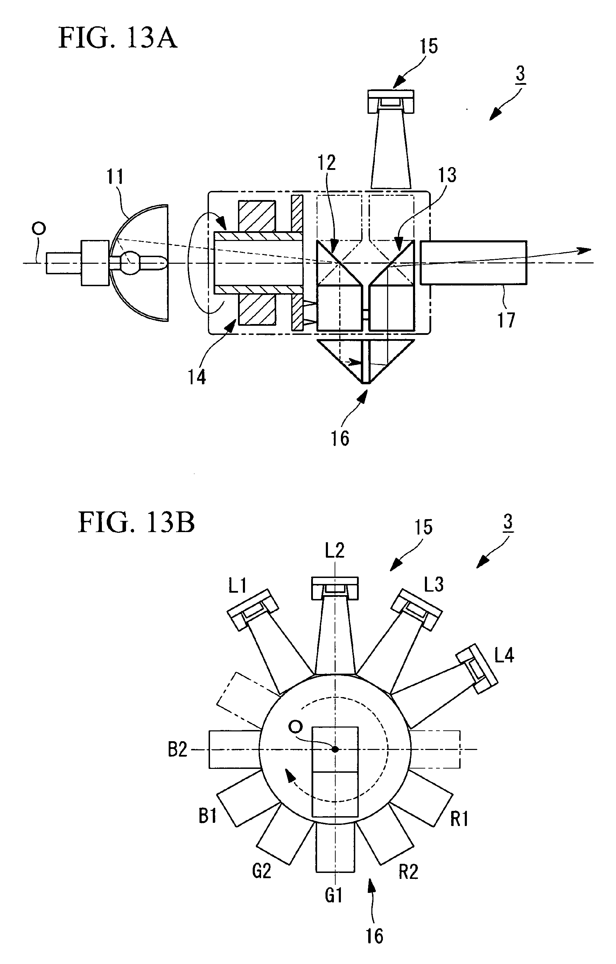

As shown in Figs. 13A and 13B, in an illumination device 3 according to this embodiment, a plurality of semiconductor light source units 15 denoted by the reference signs L1, L2, L3, and L4 and a plurality of wavelength-range selecting units 16 denoted by the reference signs R1, R2, G1, G2, B1, and B2 are arrayed in the circumferential direction about the emission optical axis O. Since wavelength-range selecting units corresponding to the reference signs W1 and W2 are not provided, the lighting unit 3 is configured such that the emission rod 17 emits only some components of the white light.

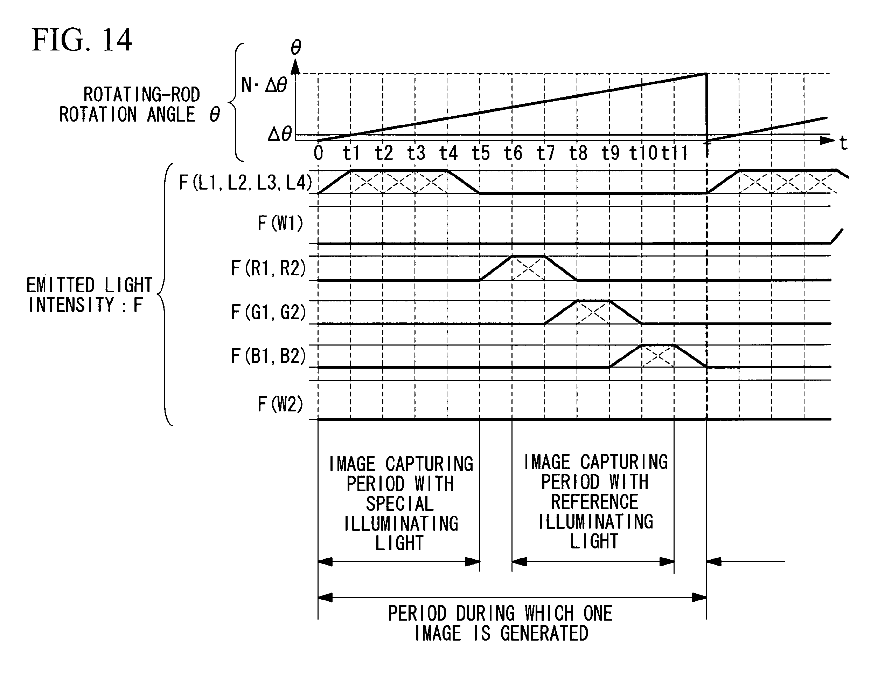

Now, the lighting sequence of the illumination device 3 according to this embodiment will be described using Fig. 14.
As shown in Fig. 14, when the first light guiding unit 12 and the second light guiding unit 13 come to the position facing the semiconductor light source units 15 denoted by the reference signs L1 to L4, i.e., at time 0 to t4, the semiconductor light source units 15 denoted by the reference signs L1 to L4 are sequentially turned on in a pulsed manner, so that the intensity F(L1, L2, L3, L4) of light emitted from the emission rod 17 is constant.
Then, as the first light guiding unit 12 and the second light guiding unit 13 rotate from the position facing the wavelength-range selecting unit 16 corresponding to the reference sign R1 (time t6) to the position facing the wavelength-range selecting unit 16 corresponding to the reference sign B2 (time t11), light components that have passed through the wavelength-range selecting units 16 corresponding to the reference signs R1 to B2 are sequentially emitted from the emission rod 17.

Here, time 0 to t5, in which illuminating light is emitted from the semiconductor light sources L1 to L4, serves as an image capturing period with special illuminating light for an image capturing unit (not shown), and time t6 to t11, in which light components that have passed through the wavelength-range selecting units 16 corresponding to the reference signs R1 to B2 are emitted, serves as an image capturing period with reference illuminating light. Accordingly, a remaining period obtained by subtracting the image capturing period with the special illuminating light and the image capturing period with the reference illuminating light from a period during which one image is generated as a period for performing image processing on the image acquired by the image capturing unit.

### [Fourth Embodiment]

Next, an illumination device according to a fourth embodiment of the present invention will be described with reference to the drawings.
The illumination device according to this embodiment differs from the above-described embodiments in that the intensity of white light is detected, an abnormality of the white light lamp is determined based on the detected intensity, and the operation mode is switched. Hereinafter, regarding the illumination device according to this embodiment, a description of commonalities with the above-described embodiments will be omitted, and the description will be directed mainly to differences.

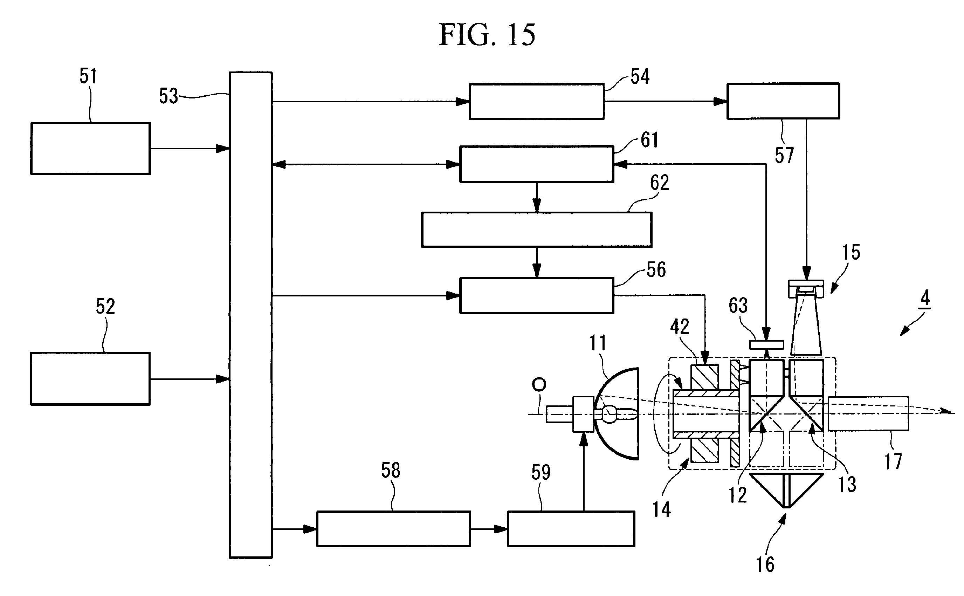

As shown in Fig. 15, an illumination device 4 according to this embodiment includes a light sensor (light detecting unit) 63 that is disposed radially outward of the first light guiding unit 12 and that detects the intensity of white light guided radially outward by the first light guiding unit 12, a light-sensor driver 61 that drives the light sensor 63, and a fail-safe monitoring controller 62 that determines an abnormality of the white light lamp 11 based on the intensity of white light detected by the light sensor 63 and switches the operation mode.

The light-sensor driver 61 is configured to drive the light sensor 63 based on a control signal from the system controller 53 and to output the intensity of white light detected by the light sensor 63 to the system controller 53 and the fail-safe monitoring controller 62.
The fail-safe monitoring controller 62 is configured to issue an instruction to the rotating-motor driver 56 so that the entrance end of the second light guiding unit 13 faces one of the semiconductor light source units 15 when the intensity of white light detected by the light sensor 63 is less than or equal to a predetermined value.

The rotating-motor driver 56 is configured to control the operation of the rotating motor 42 based on control signals from the system controller 53 and the fail-safe monitoring controller 62.
The system controller 53 is configured to constantly keep the semiconductor light source units 15 turned on when the intensity of white light detected by the light sensor 63 is less than or equal to the predetermined value.

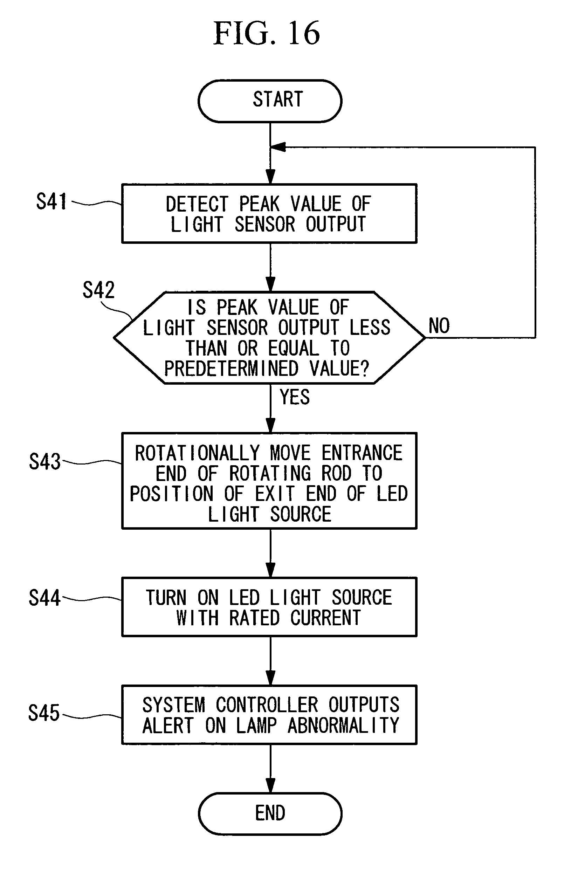

The control flow of the thus-configured illumination device 4 will be described below using Fig. 16.
Upon the start of illumination, when an output peak value of the light sensor 63 is detected (S41), it is determined whether the peak value is less than or equal to the predetermined value (S42).
When the peak value is less than or equal to the predetermined value, the system controller 53 rotationally drives the rotating motor 42 so that the entrance end of the second light guiding unit 13 comes to the position facing the exit end of one of the semiconductor light source units 15 (S43), and the semiconductor light source unit 15 is turned on with a rated current (S44). Then, the system controller 53 reports an abnormality of the white light lamp 11 by means of a lamp, buzzer, or the like to the user (S45).

As described above, with the illumination device 4 according to this embodiment, the fail-safe monitoring controller 62 detects an abnormality of the white light lamp 11 based on a value detected by the light sensor 63, and when an abnormality is detected, it is possible to switch from the white light emitted from the white light lamp 11 to illuminating light emitted from one of the semiconductor light source units 15. Accordingly, for example, by applying the illumination device 4 to an endoscope system and providing a light guiding member (not shown) that guides the illuminating light to an observed area, an image capturing element (not shown) that acquires an image of the observed area, and a display (not shown) that displays the acquired image, even when the white light lamp 11 is burnt out, it is possible to continue endoscopic observation or the like tentatively by using the illuminating light from one of the semiconductor light source units 15.

### [Fifth Embodiment]

Next, an illumination device according to a fifth embodiment of the present invention will be described with reference to the drawings.
The illumination device according to this embodiment differs from the above-described embodiments in that a white light lamp is not employed, and illuminating light from a semiconductor light source is converted into light of predetermined colors, and the light of the predetermined colors is emitted in a switched fashion. Hereinafter, regarding the illumination device according to this embodiment, a description of commonalities with the above-described embodiments will be omitted, and the description will be directed mainly to differences.

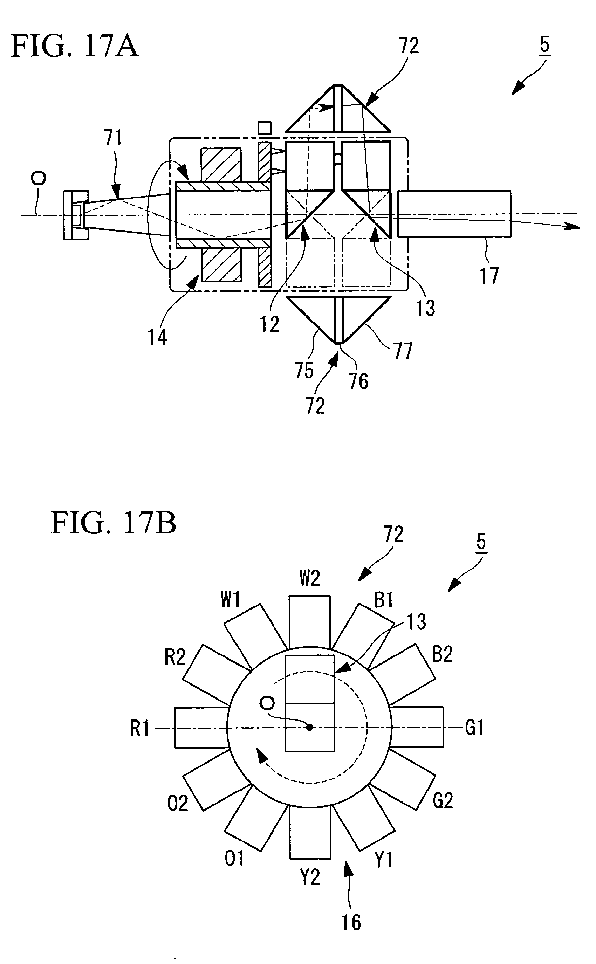

As shown in Fig. 17A, an illumination device 5 according to this embodiment includes a semiconductor light source unit 71 that emits illuminating light, a first light guiding unit 12 and a second light guiding unit 13 that are arrayed in a direction along the emission optical axis O of the semiconductor light source unit 71, a rotating unit 14 that is connected to the first light guiding unit 12 and the second light guiding unit 13 and that rotates these light guiding units together about the emission optical axis O, a plurality of wavelength conversion units 72 that are disposed outward of the first light guiding unit 12 and the second light guiding unit 13 on radially opposite sides, and an emission rod 17 that is arrayed with the second light guiding unit 13 in the direction along the emission optical axis O.

The wavelength conversion units 72 each include a first right-angled prism 75 that reflects the illuminating light guided in a radially outward direction by the first light guiding unit 12 in a direction substantially parallel to the emission optical axis O, a second right-angled prism 77 that reflects the light reflected by the first right-angled prism 75 in a radially inward direction, and a phosphor 76 that is disposed between the first right-angled prism 75 and the second right-angled prism 77 and that converts the illuminating light into light in a predetermined color. Thus, the wavelength conversion units 72 are each configured to convert the illuminating light guided in the radially outward direction into light of a predetermined color while returning the illuminating light in the radially inward direction.
Furthermore, as shown in Fig. 17B, the wavelength conversion units 72 are arrayed at twelve positions (reference signs W1, W2, R1, R2, G1, G2, B1, B2, Y1, Y2, O1, and 02) circumferentially about the emission optical axis and individually include phosphors 76 having different conversion wavelength ranges.

The illumination method of the thus-configured illumination device 5 will be described below.
As shown in Fig. 17, when the first light guiding unit 12 and the second light guiding unit 13 are located at the position facing the wavelength conversion unit 72 corresponding to the reference sign W2, illuminating light is emitted from the semiconductor light source unit 71, and the illuminating light is guided to the wavelength conversion unit 72 corresponding to the reference sign W2 by the first light guiding unit 12. Then, the illuminating light is converted into white light by the wavelength conversion unit 72 corresponding to the reference sign W2, and the white light is guided by the second light guiding unit 13 and the emission rod 17 in the direction along the emission optical axis O. Here, for example, when blue excitation light is employed as the illuminating light emitted by the semiconductor light source unit 71, white light can be obtained by using a phosphor 76 that emits yellow light.

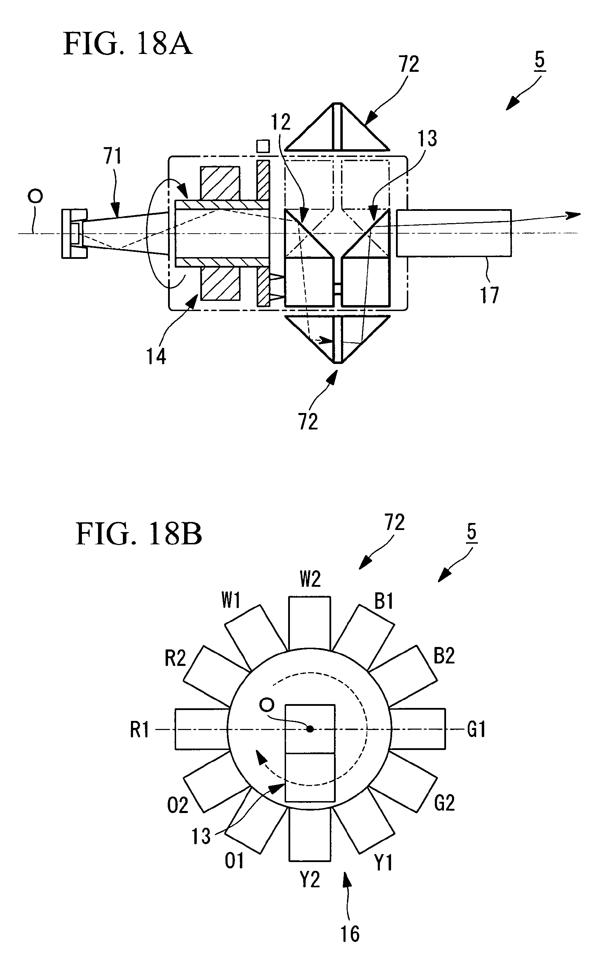

Furthermore, as shown in Fig. 18, when the first light guiding unit 12 and the second light guiding unit 13 are located at the position facing the wavelength conversion unit corresponding to the reference sign Y2, illuminating light is emitted from the semiconductor light source unit 71, and the illuminating light is guided to the wavelength conversion unit 72 corresponding to the reference sign Y2 by the first light guiding unit 12. Then, the illuminating light is converted into yellow light by the wavelength conversion unit 72 corresponding to the reference sign Y2, and the yellow light is guided by the second light guiding unit 13 and the emission rod 17 in the direction along the emission optical axis O.
As described above, by rotating the first light guiding unit 12 and the second light guiding unit 13 about the emission optical axis O by the rotating unit 14, it is possible to emit light of predetermined colors sequentially by switching.

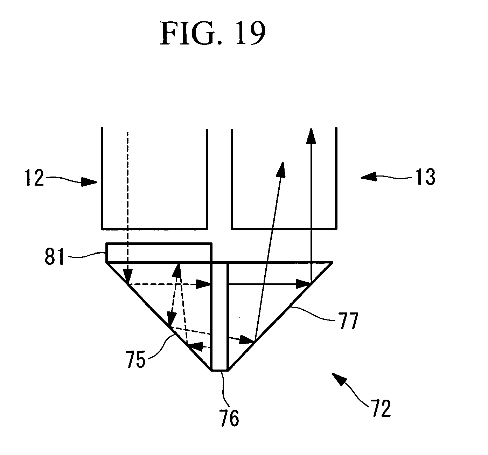

In the illumination device 5 described above, as shown in Fig. 19, a dichroic filter 81 that passes the illuminating light from the semiconductor light source unit 71 and that reflects light components in other wavelength ranges may be provided between the first light guiding unit 12 and the phosphor 76 of each of the wavelength conversion units 72.
Accordingly, radiated light that has passed through the phosphor 76 is prevented from returning to the first light guiding unit 12, so that all the excited light can be guided to the second light guiding unit.

Although the embodiments of the present invention have been described above in detail with reference to the drawings, the specific configuration is not limited to the embodiments, and design modifications or the like not departing from the spirit of the present invention are encompassed.
For example, although the semiconductor light source units 15 and the semiconductor light source unit 71 emit blue excitation light in the examples described, other colors may be adopted depending on the application of the illuminating light.

Furthermore, by applying one of the illumination devices according to the above-described embodiments to an endoscope system, and by providing a light guiding member that guides illuminating light emitted by the illumination device to an observed area, an image capturing element that acquires an image of the observed area irradiated with the illuminating light guided by the light guiding member, and a display that displays the image acquired by the image capturing element, it is possible to irradiate the observed area via the light guiding member with light components or illuminating light in a wavelength range suitable for the observed area or symptoms and to capture an image of the observed area with the image capturing element and display the image on the display. This makes it possible to improve the accuracy of special-light observation and the working efficiency.

## Claims

1. An illumination device comprising:
at least one white light source that emits white light;
a first light guiding unit that is disposed on an emission optical axis of the white light source and that guides the white light emitted from the white light source outward in a radial direction perpendicular to the emission optical axis;
a second light guiding unit that is arrayed with the first light guiding unit in a direction along the emission optical axis and that guides light going inward in the radial direction in the direction along the emission optical axis;
a rotating unit that rotationally drives the first light guiding unit and the second light guiding unit together about the emission optical axis;
a plurality of semiconductor light sources that are disposed outward of the second light guiding unit in the radial direction and arrayed in a circumferential direction about the emission optical axis and that emit illuminating light inward in the radial direction; and
a plurality of wavelength-range selecting units that are disposed outward of the first light guiding unit and the second light guiding unit on opposite sides in the radial direction at circumferential positions different from those of the semiconductor light sources and that select light components in predetermined wavelength ranges from the white light guided by the first light guiding unit and return the light components to the second light guiding unit.

2. An illumination device according to Claim 1, wherein the white light source is a xenon lamp, a halogen lamp, a metal halide lamp, or an ultra-high-pressure mercury lamp.

3. An illumination device according to Claim 1 or 2, wherein the wavelength-range selecting units each include:
a first right-angled prism that reflects the white light guided by the first light guiding unit in a direction substantially parallel to the emission optical axis;
a second right-angled prism that reflects the light reflected by the first right-angled prism inward in the radial direction; and
a wavelength-range selecting filter that passes only light components in the predetermined wavelength range.

4. An illumination device according to any one of Claims 1 to 3, wherein the first light guiding unit includes:
a right-angled prism that reflects the white light emitted from the white light source outward in the radial direction; and
a parallel rod that guides the white light reflected by the right-angled prism to the wavelength-range selecting units.

5. An illumination device according to any one of Claims 1 to 4, wherein the second light guiding unit includes:
a parallel rod that guides the light components returned by the wavelength-range selecting units and the illuminating light emitted from the semiconductor light sources inward in the radial direction; and
a right-angled prism that reflects the light components and illuminating light guided by the parallel rod in the direction along the emission optical axis.

6. An illumination device according to any one of Claims 1 to 5, further comprising:
an illumination-mode selecting unit that enables selection of an illumination mode; and
a controller that controls the turn-on and turn-off timing and light intensities of the semiconductor light sources, the turn-on and turn-off timing and light intensity of the white light source, and the operation of the rotating unit by different methods based on the illumination mode selected.

7. An illumination device according to Claim 6, wherein, in accordance with the illumination mode selected, the controller causes the rotating unit to rotate at a constant speed and causes the semiconductor light source facing the entrance end of the second light guiding unit to turn on.

8. An illumination device according to Claim 6 or 7, wherein, in accordance with the illumination mode selected, the controller stops the entrance end of the second light guiding unit at a position facing the wavelength-range selecting unit whose predetermined wavelength range includes all components of the white light.

9. An illumination device according to any one of Claims 6 to 8, wherein, in accordance with the illumination mode selected, the controller stops the entrance end of the second light guiding unit at a position facing the wavelength-range selecting unit or semiconductor light source corresponding to a specific color.

10. An illumination device according to Claim 8 or 9, wherein the controller changes the position of the second light guiding unit relative to the wavelength-range selecting units or the semiconductor light sources to adjust the intensity of light captured by the second light guiding unit.

11. An illumination device according to any one of Claims 1 to 10, further comprising:
a light detecting unit that detects the intensity of the white light guided by the first light guiding unit; and
a fail-safe monitoring controller that detects an abnormality of the white light source based on the intensity of the white light detected by the light detecting unit and, when an abnormality is detected, that keeps the semiconductor light sources constantly turned on and causes the entrance end of the second light guiding unit to face the semiconductor light sources.

12. An endoscope system including an illumination device according to any one of Claims 1 to 11, comprising:
a light guiding member that guides illuminating light emitted by the illumination device to an observed area;
an image capturing element that acquires an image of the observed area irradiated with the illuminating light guided by the light guiding member; and
a display that displays the image acquired by the image capturing element.
